# EUROPEAN PATENT APPLICATION

(11) **EP 4 335 387 A1**
(43) Date of publication of application: **13.03.2024**
(21) Application number: 22315208.3
(22) Date of filing: 09.09.2022
(51) Int. Cl.: A61B 17/00

(54) **ENDOLUMINAL DEVICE AND METHOD FOR CLOSING AN OPENING IN A TUBULAR STRUCTURE OF A PATIENT**

(71) Applicant: Caranx Medical SAS, 75013 Paris (FR)
(72) Inventor: CERRUTI, Giulio, 06700 Saint-Laurent-Du-Var (FR); SEJOR, Eric, 06000 Nice (FR); SMITS, Jonas Victor Harmen, 3360 Bierbeek (BE); BERTHET-RAYNE, Pierre, 06800 Cagnes-sur-Mer (FR)
(74) Representative: Hepp Wenger Ryffel AG

(57) **Abstract**

The invention provides an endoluminal device 10 for closing an opening 61 in a tubular structure 60 of a patient, preferably a vessel. The device is axially expandable in at least one direction and comprises a tubular sleeve 20 and an anchor 30. The anchor 30 is configured to anchor the device 10 within the tubular structure 60. The tubular sleeve 20 has a first compressed configuration 22 and a second expanded configuration 22' for closing the vessel 60.

## Description

The present invention relates an apparatus and a method for closing an opening in a tubular structure of a patient according to the independent claims.

Percutaneous procedures entail the access of inner bodily structures by way of puncture of the patient's skin with a needle. Such procedures are often used in vascular surgery where the puncture site grants access to a blood vessel of the patient allowing the surgeon to navigate within the vascular system to attain the target structure. Once a puncture site had been created, other instruments, such as guidewires and introducer sheaths, are inserted through the puncture site for performing the surgery. Once the procedure finished, the inserted instruments are removed, and the puncture site must be closed so as to avoid complications such as bleeding, haemorrhages and infections.

A number of devices are known to this effect. US 201106131 shows a tubular closure device having a radially self-expandable scaffold and a tether which is positioned within the patient's vessel upstream of the puncture site. The device is then radially expanded and pulled down the vessel by the tether to close the puncture site. However, problems can occur if the tether breaks and the position of the device can no longer be changed.

The device provided by CA 2640480 has an elongate tubular body with a distal and proximal end. The ends expand when positioned to form proximal and distal wings upon rotation of the elongate member.

It is an object of the present invention to overcome the drawbacks of the prior art and in particular to provide a device that shall allow efficient, easy to use and fast sealing of punctures or incisions formed in a blood vessel.

These and other objects are solved with an apparatus and a method according to the independent claims.

The endoluminal device according to the invention is adapted for closing an opening in a tubular structure of a patient. The tubular structure is preferably a vessel of the vascular system of the patient. The device is configured to be positioned within a tubular structure of a patient.

The device may be arranged neighbouring one end of the tubular structure. The device may also be arranged neighbouring an opening in the tubular structure. For example, the device may be arranged within a vessel, preferably in the target zone that is adjacent to a puncture wound in the vessel.

The endoluminal device comprises a tubular sleeve having a longitudinal axis. The device further has at least one anchor for anchoring the device within the tubular structure of the patient. The tubular sleeve may be anchored within a vessel of the patient.

The tubular sleeve may be connected to the anchor of the endoluminal device. Optionally the anchor is connected to at least a first end of the tubular sleeve.

The tubular sleeve has a first compressed configuration for delivery to an implantation site within the tubular structure. Furthermore, the tubular structure has a second expanded configuration for closing the opening in the tubular structure of the patient.

The device is expandable in at least one direction along the longitudinal axis. Optionally the device may be expandable in at least two directions.

The device being configured to be positioned in a compressed configuration can allow for an easier minimally invasive procedure and can thus improve patient outcome and reduce the risk of complications.

Additionally, the device can allow for easier positioning of the device within a vessel of a patient given the compressed configuration. This may also allow for the opening to be closed quickly, if necessary, e.g. in case of the patient haemorrhaging.

The anchor may avoid the migrating of the device and therefore reduces the risk of complications linked to the device traveling through the vascular system.

The tubular sleeve may help ensure the opening remains closed until the opening in the vessel can heal naturally and may reduce the amount of blood loss during the healing process.

The endoluminal device may have a lumen, optionally a through lumen. The lumen may be defined by the anchor and/or by the tubular sleeve.

The device may be self-expandable along the longitudinal axis.

A self-expandable device may allow practitioners to securely and quickly close an opening in a vessel, this may help improve patient outcome.

Additionally, a self-expandable endoluminal device for closing an opening in a tubular structure may help reduce the risk of post-procedural complications and risks of haemorrhages when used in bigger vessels such as arteries.

The components of the endoluminal device may be made of an at least partly elastic biocompatible material. Additionally or alternatively, the endoluminal device may be a temporary device made of a bioresorbable material.

The tubular sleeve may be axially expandable or axially self-expandable. Additionally or alternatively, the tubular sleeve may be displaceable away from the anchor in at least one direction. Optionally the tubular sleeve may be displaceable away from the anchor in two directions.

In other words, the tubular sleeve may be configured to longitudinally expand and/or be displaced away from the anchor of the endoluminal device in one or multiple directions.

The anchor may be radially expandable to engage with a portion of the tubular structure. Optionally, the anchor may be self-expandable to engage with a portion of the tubular structure.

The anchor may comprise a self-expandable component, e.g a self-expandable stent or a preloaded spring for engaging with the tubular structure. Alternatively or additionally, the anchor may be adapted to radially expand due to an inflatable balloon, e.g. a balloon catheter.

In other words, the anchor may be configured to be radially expanded or to radially self-expand so as to anchor the tubular sleeve to the tubular structure of the patient, e.g. anchoring the tubular sleeve in a vessel.

The device may comprise at least one deployment mechanism for expanding the device. The actuation of the deployment mechanism may induce axial expansion of the device. Additionally or alternatively, actuation of the mechanism may induce radial expansion of the endoluminal device or radial expansion of a portion of the endoluminal device, especially of the anchor.

The deployment mechanism may be attached to the sleeve and/or the anchor.

The deployment mechanism may comprise a spring, an impingement surface on which a fluid can impinge, a constriction member, at least one pulling member or combinations thereof. The deployment mechanism may be attached to or arranged on the tubular structure at an area distant from the anchor.

The device may be expanded by impact of a fluid on the impingement surface.

The pulling member may be a cable-like element configured to facilitate the expansion of the device. Optionally, the pulling member may be configured to guide the expansion of the device.

The constricting member may circumscribe at least a portion of the device to maintain the device in a compressed configuration. The constricting member may be detachable from the device for expanding the device and/or detachable once the device is expanded. For example, the contracting member, e.g. a cable, may be configured to break away from the device, e.g. by pulling on the cable or by applying a heat or a current to the cable.

Optionally, the deployment mechanism may be connected to the anchor.

In some embodiments the impingement surface may be resorbable. The impingent surface may be made of a bioresorbable material.

Providing the device with a resorbable impingement surface and/or an impingement surface that may partly reduce the cross-section of the lumen of the tubular sleeve may facilitate an automatic expansion of the device and help to quickly restore blood flow within the vessel. Additionally using the force of the blood flow and an impingement surface to expand the device avoids the need for additional elements, thus reducing costs but also the risk of the additional element(s) being faulty or detaching from the device within the vessel which could potentially cause complications during the procedure.

In some embodiments the impingement surface may at least partially reduce the cross-section of a lumen defined by the tubular sleeve.

Reducing the cross-section of the lumen provides a resistant surface against which the blood flow will push. The force of the blood flow against the impingement surface extends the device. The anchor and the impingement surface may be at least partly distanced from one another along the tubular sleeve. Optionally, the anchor and the impingement surface may be arranged at opposing extremities of the tubular sleeve.

The device may comprise an interface for operative connection with a control unit for controlling the deployment and/or expansion of the device.

In a second aspect, the invention provides a system having a device as described above and a control unit. The control unit of the system are configured to actuate the deployment mechanism.

In a third aspect the invention provides a method of closing an opening in a tubular structure of a patient using an endoluminal device, optionally an endoluminal device as described above. The method may use a system as described above.

The endoluminal device is introduced through an opening in a tubular structure of the patient. The device may be introduced within the tubular structure by an instrument.

The device, in particular the tubular sleeve, is then axially expanded within the tubular structure near the opening in the tubular structure.

The device is also anchored to the tubular structure. The device is preferably anchored at, or near, the opening in the tubular structure in such a manner that the opening of the tubular structure is not closed.

The device can be anchored to the tubular structure by radial expansion of the anchor.

The device is axially expanded to close the opening in a tubular structure of a patient. In particular, the tubular sleeve of the device axially expands to close the opening.

The steps of expansion and anchoring of may be performed interchangeably.

In other words, the device may be expanded within the tubular structure prior to or after the anchoring of the device.

The invention will be better understood with reference to the following decryption of preferred embodiments and the accompanying drawings, which show:
- Fig. 1: a schematic longitudinal cross-section view of a first embodiment of an endoluminal device for closing an opening in a tubular structure of a patient.
- Fig. 2: a schematic longitudinal cross-section view of a second embodiment of an endoluminal device for closing an opening in a tubular structure of a patient in an axially compressed configuration.
- Fig. 3: a schematic longitudinal cross-section view of a third embodiment of an endoluminal device for closing an opening in a tubular structure of a patient.
- Fig. 4: a schematic longitudinal cross-section view of a fourth embodiment of an endoluminal device for closing an opening in a tubular structure of a patient.
- Fig. 5: a schematic longitudinal cross-section view of a fifth embodiment of an endoluminal device for closing an opening in a tubular structure of a patient.
- Fig. 6: a schematic longitudinal cross-section view of a sixth embodiment of an endoluminal device for closing an opening in a tubular structure of a patient.
- Fig. 7: a schematic longitudinal cross-section view of an alternative embodiment of an endoluminal device for closing an opening in a tubular structure of a patient.
- Fig. 8: a schematic longitudinal cross-section view of an alternative embodiment of an endoluminal device for closing an opening in a tubular structure of a patient.
- Fig. 9: a schematic front view of an embodiment of an anchor of an endoluminal device for closing an opening in a tubular structure of a patient.
- Fig.: 10a-b a schematic front view of a deployment mechanism of an anchor of an endoluminal device for closing an opening in a tubular structure of a patient.
- Fig.: 11a-b a schematic longitudinal cross-section views of an alternative embodiment of an endoluminal device for closing an opening in a tubular structure of a patient.
- Fig.: 12a-d a schematic diagram illustrating steps in an example technique for closing an opening in a tubular structure of a patient with an endoluminal device.

In the illustrations, the same reference numerals are used to denote the same or equivalent features amongst different embodiments and examples. Unless described to the contrary, the description of a feature in one embodiment may also apply to the same or equivalent feature in one embodiment or example. Features may also be interchanged in embodiments as desired.

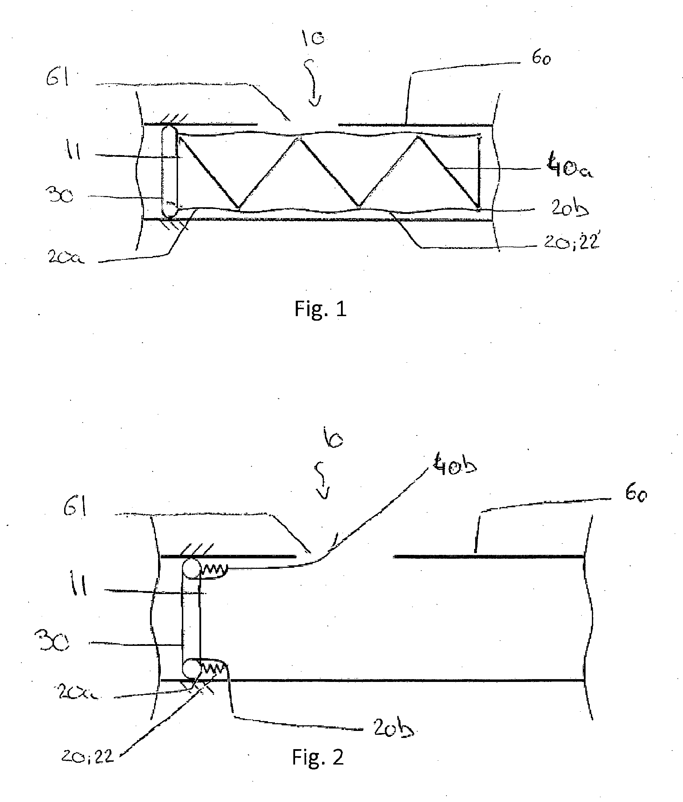

Referring to figure 1 an endoluminal device 10 is positioned within a vessel 60 of a patient for closing an opening 61 of the vessel 60. The device 10 comprises a tubular sleeve 20 having a first extremity 20a and second extremity 20b. The tubular sleeve 20 defines a lumen 11 extending from the first extremity 20a to the second extremity 20b. The tubular sleeve 20 is configured to allow the unhindered passage of fluid, e.g. blood, through the lumen 11 whilst also allowing the closing of the opening 61 in the vessel 60.

The device 10 has an anchor 30 positioned in the vicinity of the opening 61 of the vessel 60. The anchor 30 has an annular body 32 and is configured to anchor the tubular sleeve 20 within the vessel 60. In other words, the anchor 30 may avoid the migration of the device 10 within the vessel 60.

In Fig. 1, the sleeve 20 is in an expanded configuration 22' and the anchor 30 is positioned at the first end 20a of the tubular sleeve 20.

The device 10 further has a deployment mechanism in the form of a preloaded spring 40a for expanding the device within the tubular structure, e.g. within the vessel 60. The preloaded spring 40a facilitates the expansion of the device 10 from a compressed configuration 22 (see figures 2, 8 and 11a) to the expanded configuration 22'.

The preloaded spring 40a is connected to the first end 20a and to the second end 20b of the sleeve 20. Once positioned within the patient near the opening 61 of the vessel 60, the preloaded spring 40a will expand the sleeve to the expanded configuration 22' .

The expansion of the sleeve 20 allows the closing of the opening 61 in the vessel 60 of a patient.

Shown in figure 2, the device 10 is introduced within the vessel 60 of the patient in an axially compressed configuration 22. The device 10 may be passed through the opening 61 into the vessel 60.

The device 10 is anchored upstream of the opening 61 by the anchor 30 of the device 10.

The anchor 30 can be a stent adapted to radially expand to engage with the tubular structure 60 of the patient. For example, the anchor could be a self-expandable stent, or a stent adapted to be expanded by a balloon catheter.

The deployment mechanism has at least one constriction member in the form of a cable. 40b configured to maintain the tubular sleeve 20 in the axially compressed configuration 22. The cable 40b envelop at least part of the tubular structure and optionally envelops the anchor 30 to secure the sleeve 20 in an axially compressed configuration 22.

Actuation of the cable 40b may induce the self-expansion of the tubular sleeve 20. The tubular sleeve 40b can expand due to the elasticity of the sleeve 20. Optionally, the tubular sleeve 20 may have a pre-loaded spring 40a (see fig. 1) that expands after actuation of the cable 40b.

The cable 40b can be actuated by pulling and/or breaking of the cable.

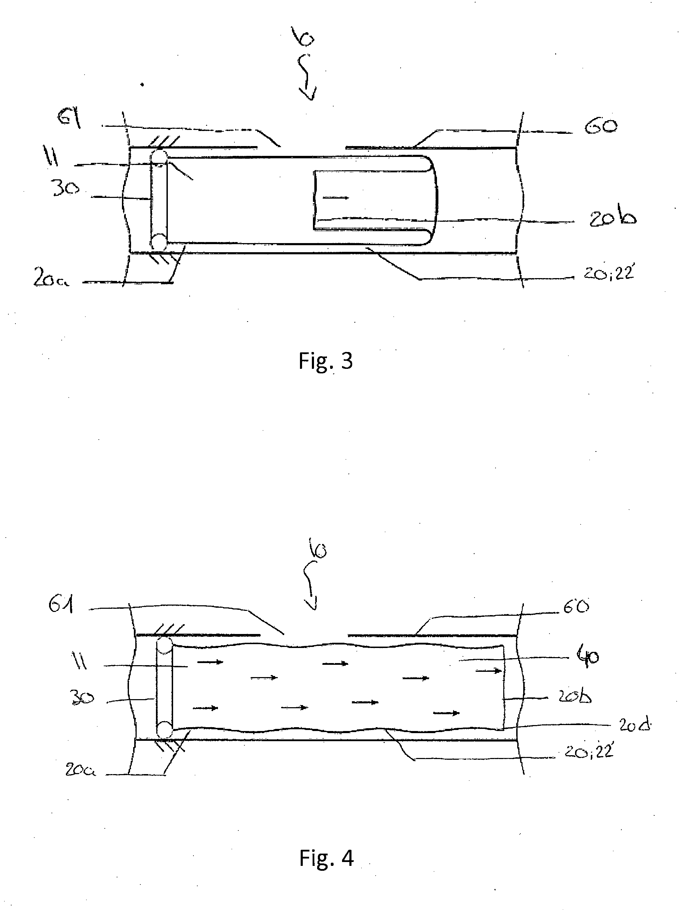

Fig. 3 shows a third embodiment of a device 10 with a sleeve 20 which is partly folded on itself. The sleeve 20 is configured to evert into the axially extended configuration. For example, the sleeve can evert due to intrinsic elasticity of the sleeve 20 or by an external force applied to the sleeve 20 or to a portion of the sleeve 20.

The sleeve 20 can optionally evert due to intrinsic elasticity of the sleeve.

The device 10 may comprise a control unit (not shown) configured to induce the eversion of the sleeve 20 upon operation of the control unit.

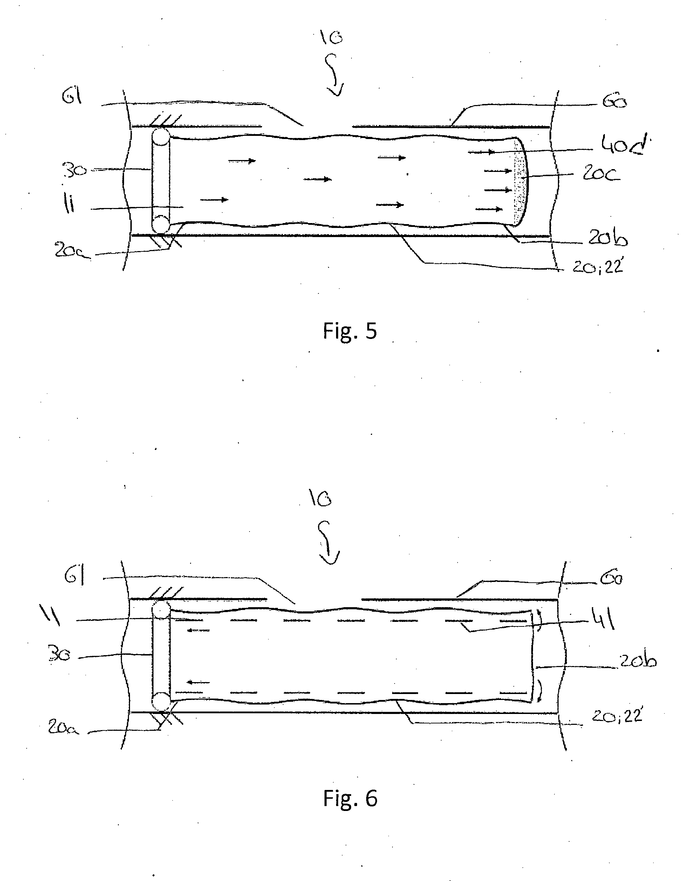

Illustrated in figures 4 and 5, a device 10 is shown in an expanded configuration. A tubular sleeve 20 is positioned in a vessel 60 of a patient in an axially expanded configuration with an anchor 30 anchored upstream of an opening 61 of the vessel 60.

In figures 4 and 5 the device 10 is also anchored within a vessel 60 of the patient by the anchor 30. The tubular sleeve 20 is configured to axially expand under a force 40d applied to an impingement surface 20d, 20c of the sleeve 20 by the blood flow through the lumen 11 impinging on the walls 20d of the sleeve 20 and inducing the expansion of the sleeve from the compressed configuration 22 (see figures 2, 8 and 11a) to the expanded configuration 22' .

The impingement surface is formed by an internal wall 20d of the tubular sleeve 20. Passage of blood through the lumen 11 of the tubular sleeve 20 causes the sleeve to expand from the compressed configuration 22 to the expanded configuration 22'.

In the alternative embodiment of figure 5 an impingement surface 20c closes the lumen 11 at one end 20b of the tubular sleeve 20. The impingement surface 20c positioned at an end 20b of the sleeve 20 allows the expansion of the sleeve 20 under the impact of the blood flow 40d on the impingement surface 20c.

The impingement surface 20c in figure 5 is made of a bioresorbable material to allow restitution of blood flow through the lumen 11 once the sleeve 20 has expanded to close the opening 61 of the vessel 60.

Illustrated in figure 6, a device 10 is again anchored upstream of the opening 61 in the vessel 60 of the patient by an anchor 30. Here the deployment mechanism has pulling members, e.g. expansion wires 41. The expansion wires 41 of figure 6 are connected to one end 20a of the tubular sleeve 20 and extend along the length of the tubular sleeve 20. The expansion wires 41 are connected to the second end 20b of the tubular sleeve 20. The expansion wires can further be used to steer the device, e.g. during deployment.

The expansion wires 41 can optionally be integrated within the wall of the tubular sleeve and/or attached to the surface of the tubular sleeve 20.

The expansion wires 41 are configured to steer an expansion of the tubular device 20. For example, the expansion wire may assist the correct deployment of the device expanding due to propelling forces, e.g. blood impinging on an impingement surface.

The expansion wire may help avoid the incorrect deployment of the device and therefore reduce the risk of complications during the procedure.

The expansion wires 41 can be operated by a control unit (not shown) or be operated manually for the expansion of the device 10.

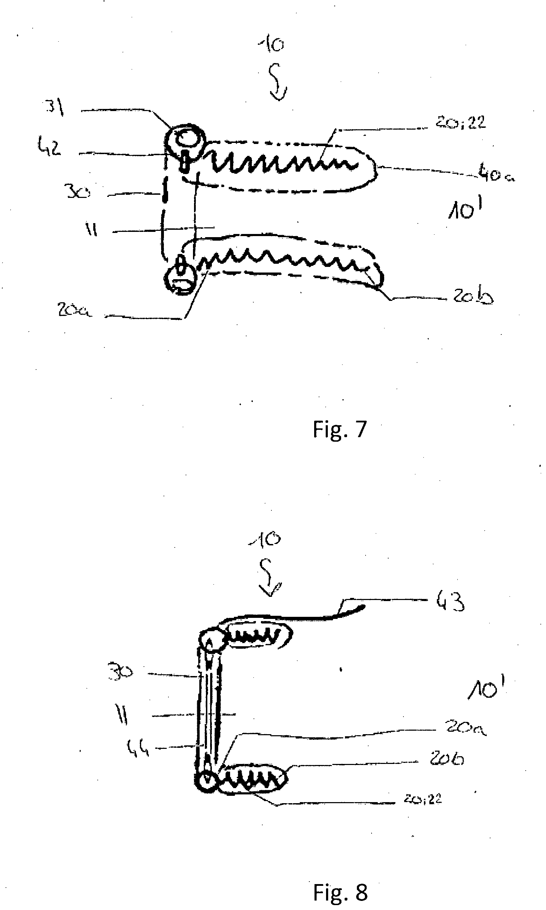

Fig.7, 8 and 9 show three different embodiments for release mechanisms, in particular retaining elements which are released by inflation of a chamber, retaining elements which are released by pulling an elastic element or retaining elements which are released by pulling latches.

Referring to figure 7, the device 10 is in a compressed configuration 10'. An anchor 30 of a device 10 has an internal chamber 31. The internal chamber 31 is configured to inflate so as to radially expand the anchor 30. The radial expansion of the anchor 30 secures the position of the device within the vessel 60 of the patient. Optionally, the radial expansion of the anchor 30 can induce the expansion of the device 10. Additionally or alternatively, the radial inflation of the anchor may induce the release of a constriction member 42.

The sleeve 20 is in an axially compressed configuration 22. The deployment mechanism comprises one or more constricting members 42. The constricting members comprises at least one fastening feature, e.g. plug-like elements 42, optionally attached to at least one constricting element (e.g. cable) (see fig. 2). The plugs 42, and optionally the constricting element, are configured to maintain the sleeve 20 in a compressed configuration 22.

The anchor 30 has a plurality of apertures around its circumference. The plugs 42 of figure 7 are configured to be inserted into and removed from the apertures of the anchor 30. Inflation of the internal chamber 31 is configured to release, for example by pushing, the plug-like elements 42 from the apertures. This releases the constricting element and releases the sleeve 20. Disengaging the sleeve in the compressed configuration induces the expansion of the tubular sleeve 20 from a compressed configuration 22 to the expanded configuration 22' thus closing the opening 61 of the vessel 60 of the patient.

Alternatively, the plug-like elements 42 may also be disengaged from the apertures of the anchor 30 by one or more pulling cables (not shown). The pulling cable(s) may pull the fastening features 42, e.g. plugs, from the apertures thus allowing the expansion of the tubular sleeve 20.

Shown in figure 8 the deployment mechanism 44,43 comprises a constricting member. Here an elastic ring 44 is configured to keep retaining elements in place thereby maintaining the device 10 in a compressed state 10'. By pulling the elastic ring 44, the retaining elements are freed. The ring 44 is configured be attached at least a part of the device 10, e.g. the anchor 30 and/or the tubular sleeve 20.

The device 10 has a pulling member, e.g. a release cable 43, configured to remove, e.g. by pulling, the ring 44 from the device 10. Removing the ring 44 induces the expansion of the device 10 to the expanded configuration 22'.

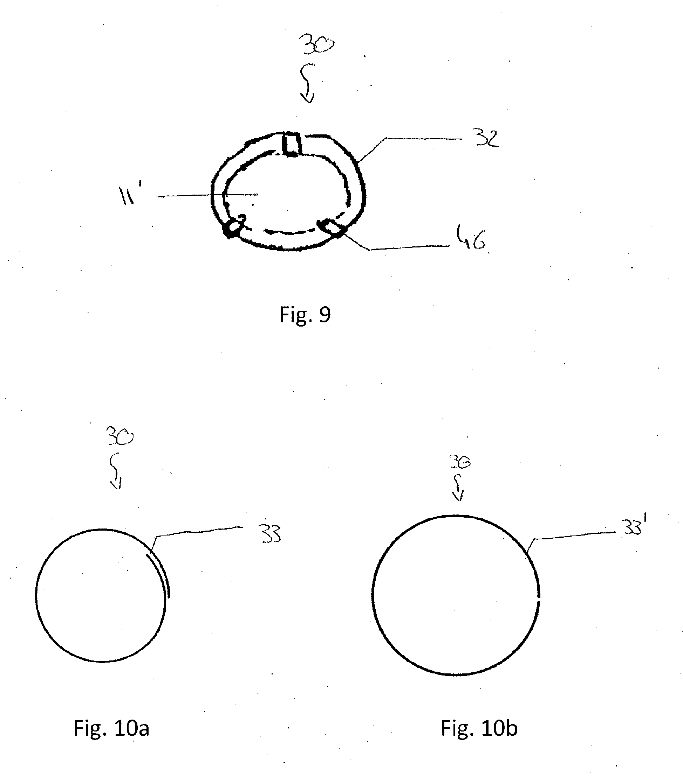

Figure 9 illustrates the anchor 30 which has an at least partly annular body 32. The circular body 32 defines a lumen 11'. The anchor lumen 11' is configured to at least partly align with the lumen 11 of the tubular sleeve 20 (see figures 1-8). The deployment mechanism 46 comprises a constricting member, here a plurality of clips 46 and one or more pulling members, here release cables (not shown in figure 9). The clips 46 are positioned around the circumference of the anchor 30, e.g. positioned at 120° with regards to one another.

The clips 46 are loaded onto the anchor 30 by longitudinally compressing the tubular sleeve 20 into the compressed configuration 22. Once the tubular sleeve 20 is compressed, the clips 46 attach to the anchor 30 to maintain the sleeve 20 in a compressed configuration (cf Figs. 7 and 8).

In this embodiment, a release cable (not shown) is configured to rotate the clips 46 around the centre of the anchor lumen 11' to expand the device 10 from the compressed configuration to the expanded configuration.

Referring to figures 10a and 10b, the annular body 32 of the anchor (see figure 1 and 9) can be at least partly elastic and configured to axially expand from a compressed state 33 to an expanded state 33'. The anchor 30 can be made of an elastic material and/or can host a preloaded spring to induce the expansion.

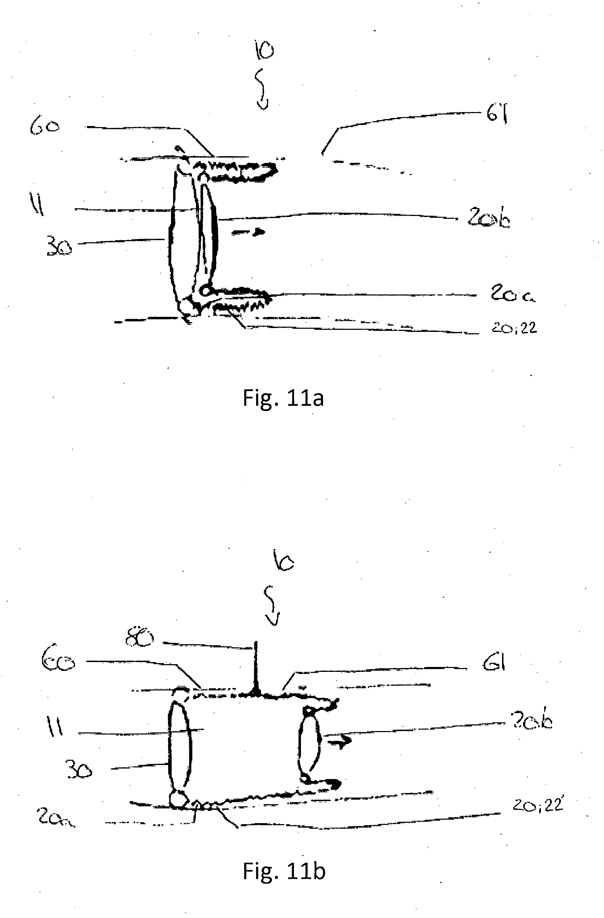

Shown in figures 11a and 11b, the axially compressed device 10 (fig. 11a) is positioned within the vessel 60 of the patient. The device 10 is anchored near an opening 61 of the vessel 60 by the anchor 30.

The tubular sleeve has a lumen 11 and is attached to the anchor at the first end 20a of the tubular sleeve. The tubular sleeve is in a compressed configuration 22 (figure 11a). In the compressed configuration 22 the tubular sleeve 20 is folded in on itself in such a manner that the ends of the tubular sleeve 20a and 20b are at least partly adjacent with regards to one another.

In figure 11b the tubular sleeve 20 longitudinally extends away from the first end 20a of the sleeve, e.g. due to the force of blood flow on the walls 20d of the tubular sleeve 20. The extension of the second end 20b of the tubular sleeve 20 can be assisted by an external instrument 80 placed at the opening 61 of the vessel 60 and in contact with the device 10. The external instrument 80 is configured to avoid the device 10 from extending through the opening 61 of the vessel 60 as it extends. For example, the instrument 80 can apply pressure the device 10, e.g. by using a fluid injected at the opening 61. This helps to reduce the risk of the device 10 escaping through the opening as it extends within the vessel.

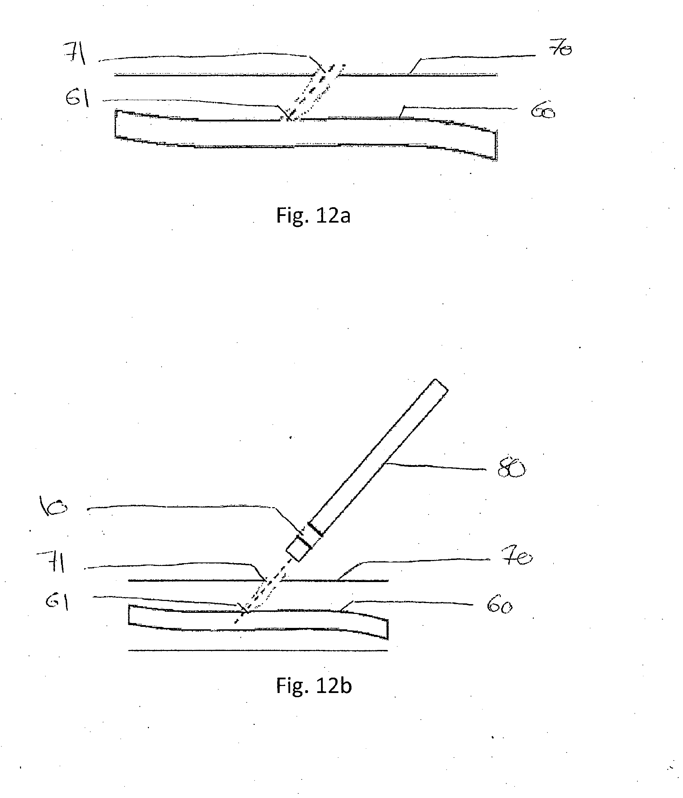

Illustrated in figures 12a-12d are the schematic stages of a method of closing the opening 61 of the vessel 60 in a patient using the endoluminal device 10.

Figure 12a shows a wound in a patientextending from an opening 61 of a vessel 60 to an opening 71 of the skin 70 of the patient. The wound can be a puncture wound for example.

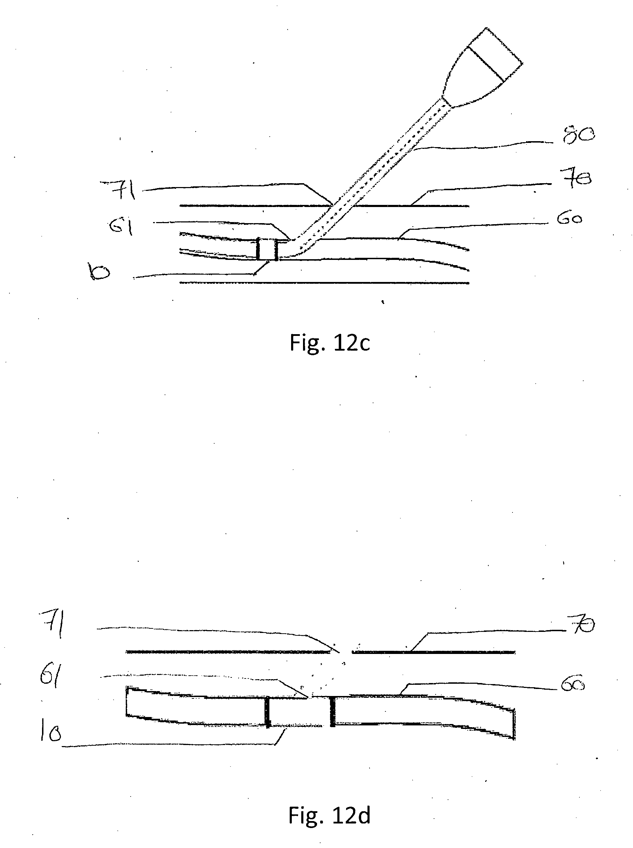

In figure 12b and 12c an instrument 80 is used to introduce the endoluminal device 10 in a radially and axially compressed configuration within the vessel 60 of a patient. The instrument 80 and endoluminal device 10 are introduced through an opening 71 in the skin 70 of the patient and through an opening 61 of a vessel 60.

In figure 12d the endoluminal device 10 is radially expanded to the expanded configuration 22' and closes off the vessel opening 61. Blood is thus blocked from escaping the vessel and blood flow through the vessel 60 is restored.

## Claims

1. Endoluminal device (10) for closing an opening in a tubular structure 60 of a patient, preferably an opening (61) in a vessel (60), the device (10) comprising a tubular sleeve (20) having a longitudinal axis and at least one anchor (30) for anchoring the device (10) within the tubular structure (60), optionally arranged neighbouring one end of the tubular structure (60), wherein
the tubular sleeve (20) has a first compressed configuration (22) for delivery to an implantation site within the tubular structure (60) and a second expanded configuration 22' for closing the opening (61) and
the device (10) is expandable in at least one direction along the longitudinal axis, optionally expandable in at least two directions.

2. Device according to claim 1, wherein the device (10) is self-expandable along the longitudinal axis.

3. Device according to any claim 1 or 2, wherein the tubular sleeve (20) is axially expandable.

4. Device according to any claim 1 to 3, wherein the tubular sleeve (20) is axially expandable or displaceable away from the anchor (30) in at least one direction, optionally in two directions.

5. Device according to any claim 1 to 4, wherein the anchor (30) is radially expandable, preferably self-expandable, to engage with a portion of the tubular structure 60.

6. Device according to any claim 1 to 5, wherein the device (10) comprises at least one deployment mechanism (40a; 40b; 41; 42; 43; 44; 46; 20c; 20d) for expanding the device (10), preferably wherein actuation of the deployment mechanism (40a; 40b; 41; 42; 43; 44; 46; 20c; 20d) induces axial and/or radial expansion of the device (10), and optionally wherein the deployment mechanism (40a; 40b; 41; 42; 43; 44; 46; 20c; 20d) is attached to the sleeve (20) and/or the anchor (30).

7. Device according to claim 6, wherein deployment mechanism (40a; 40b; 41; 42; 43; 44; 46; 20c; 20d) comprises one or a combination of the following: a spring (40a); an impingement surface for a fluid (20d; 20c), a constricting member (40b; 42; 44; 46) at least one pulling member (40b;43) attached to the tubular structure (20) at an area distant from the anchor (30).

8. Device according to any claim 1-7, wherein the impingement surface (20d;20c) is resorbable.

9. Device according to any claim 1-8, wherein the impingement surface (20d;20c) at least partially reduces the cross-section of a lumen (11) defined by the tubular sleeve (20).

10. Device according to any claim 7 to 9, wherein the anchor (30) and the impingement surface (20d; 20c) are at least partly distanced from one another along the tubular sleeve (20),and are optionally arranged at opposing extremities (20a; 20b) of the tubular sleeve (20).

11. Device according to any claim 1 to 10, wherein the device (10) comprises an interface for operative connection with a control unit for controlling the deployment and/or expansion of the device (10).

12. A system having a device (10) according to claim 11 and a control unit, wherein the control unit is configured to actuate the deployment mechanism (40b; 41; 42; 43; 44; 46).

13. Method of closing an opening (61) in a tubular structure (60) of a patient using an endoluminal device (10), optionally according to any claim 1 to 12, wherein the method comprises the steps of:
(i) introducing the endoluminal device (10) through an opening (61) into a tubular structure (60) of the patient;
(ii) radially expanding the device (10) within the tubular structure (60) near the opening (61) in the tubular structure (60);
(iii) anchoring the device (10) to the tubular structure (60), preferably at, or near, the opening (61) in the tubular structure (60);
(iv) axially expanding the device (10) to close the opening (61) in a tubular structure (60) of a patient.
